(19) Europäisches Patentamt
European Patent Office
Office européen des brevets

(11) EP 4 534 201 A1

(12) **EUROPEAN PATENT APPLICATION**
published in accordance with Art. 153(4) EPC

(43) Date of publication:
**09.04.2025 Bulletin 2025/15**

(21) Application number: **24827786.5**

(22) Date of filing: **04.07.2024**

(51) International Patent Classification (IPC):
***B01J 31/02*** *(2006.01)*

(52) Cooperative Patent Classification (CPC):
**B01J 31/02; B01J 31/04; B01J 37/04; B01J 38/48; C07C 37/055; C07C 37/70; C07C 39/16; C08J 11/28;** Y02W 30/62

(86) International application number:
**PCT/KR2024/009452**

(87) International publication number:
**WO 2025/028825 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.07.2023 KR 20230099505**

(71) Applicants:
- **LG Chem, Ltd.**
  **Yeoui-daero, Youngdungpo-gu**
  **Seoul 07336 (KR)**
- **Korea University Research and Business Foundation**
  **Seoul 02841 (KR)**

(72) Inventors:
- **LEE, Jeongbin**
  **Daejeon 34122 (KR)**
- **HONG, Mooho**
  **Daejeon 34122 (KR)**
- **SEO, Jihun**
  **Seoul 02841 (KR)**
- **SEO, Sojung**
  **Seoul 02841 (KR)**
- **LEE, Seunghun**
  **Seoul 02841 (KR)**
- **LEE, Eunji**
  **Seoul 02841 (KR)**
- **LEE, Joonbum**
  **Seoul 02841 (KR)**

(74) Representative: **Goddar, Heinz J.**
**Boehmert & Boehmert**
**Anwaltspartnerschaft mbB**
**Pettenkoferstrasse 22**
**80336 München (DE)**

# (54) CATALYST FOR DEPOLYMERIZATION OF POLYCARBONATE-BASED RESIN, METHOD FOR PREPARING SAME, METHOD FOR DECOMPOSING POLYCARBONATE-BASED RESIN USING SAME, AND METHOD FOR RECOVERING CATALYST FOR DEPOLYMERIZATION OF POLYCARBONATE-BASED RESIN

(57) The present disclosure relates to a catalyst for depolymerizing a polycarbonate-based resin comprising a compound represented by Chemical Formula 1, a preparation method thereof, and a decomposition method of a polycarbonate-based resin, and a recovery method of a catalyst for depolymerizing a polycarbonate-based resin.

EP 4 534 201 A1

**Description**

**[TECHNICAL FIELD]**

CROSS-REFERENCE TO RELATED APPLICATION(S)

This application claims the benefit of Korean Patent Application No. 10-2023-0099505 filed on July 31, 2023 with the Korean Intellectual Property Office, the disclosure of which is incorporated herein by reference in its entirety.

The present disclosure relates to a catalyst for depolymerizing a polycarbonate-based resin, a preparation method thereof, and a decomposition method of a polycarbonate-based resin, and a recovery method of a catalyst for depolymerizing a polycarbonate-based resin.

**[BACKGROUND]**

Polycarbonate is a plastic that has high heat resistance, impact resistance, and transparent optical properties, and thus is used in a wide range of fields, including not only industrial products such as various optical products, electronic products, building materials, but also daily necessities such as water bottles and tableware.

Polycarbonate is often produced so as to have a variety of colors and physical properties depending on the field of use, and thus, physical recycling methods are inappropriate, which has the limitation in that even recycled plastics produced thereby show a relatively large deterioration in physical properties.

The depolymerization process is a chemical recycling method that recovers polycarbonate waste as bisphenol A which is a raw material, and then reuses it for plastic production, which is a recycling method that not only can overcome the deterioration of physical properties that occurs in physical recycling, but also can reduce the production of additional bisphenol A.

Conventional depolymerization processes use an acid or base catalyst in the depolymerization of polycarbonate, but the acid or base catalyst have problems in that it corrodes the equipment and requires wastewater treatment and neutralization processes within the process. In addition, the acid or base catalyst has disadvantages in that the reaction is terminated by a neutralization reaction, and the catalyst cannot be recovered, thus making it uneconomical.

Therefore, there is a need to develop a novel catalyst that can replace the acid or base catalyst that has been conventionally used.

**[DETAILED DESCRIPTION OF THE INVENTION]**

**[Technical Problem]**

It is an object of the present disclosure to provide a catalyst for depolymerizing a polycarbonate-based resin and a preparation method thereof, which can secure an aromatic diol compound in a high yield by recycling a polycarbonate-based resin through chemical decomposition, and can improve chemical decomposition reactivity and reaction efficiency.

It is another object of the present disclosure to provide a decomposition method of a polycarbonate-based resin by using the catalyst for depolymerizing a polycarbonate-cased resin, and a recovery method of a catalyst for depolymerizing a polycarbonate-based resin.

**[Technical Solution]**

In order to achieve the above objects, provided herein is a catalyst for depolymerizing a polycarbonate-based resin, comprising a compound represented by the following Chemical Formula 1:

[Chemical Formula 1]

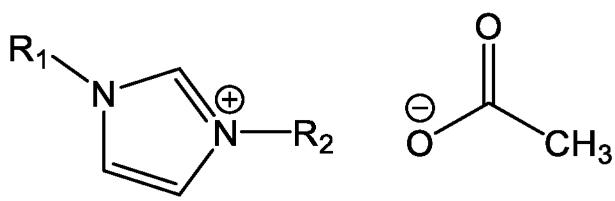

wherein in Chemical Formula 1, $R_1$ and $R_2$ are a straight chain alkyl having 1 to 3 carbon atoms.

Also provided herein is a preparation method of a catalyst for depolymerizing a polycarbonate-based resin, the method comprising the steps of: reacting 1-alkyl-imidazole and alkyl halide to prepare a compound represented by the following Chemical Formula 4; and reacting the compound represented by Chemical Formula 4 with an alkali metal acetate:

[Chemical Formula 4]

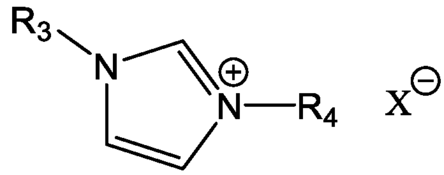

wherein in Chemical Formula 4, $R_3$ and $R_4$ are a straight chain alkyl having 1 to 3 carbon atoms, and X is halogen.

Further provided herein is a decomposition method of a polycarbonate-based resin, the method comprising subjecting a polycarbonate-based resin to depolymerization reaction in the presence of the catalyst for depolymerizing a polycarbonate-based resin.

Further provided herein is a recovery method of a catalyst for depolymerizing a polycarbonate-based resin, the method comprising the steps of: subjecting a polycarbonate-based resin to depolymerization reaction in the presence of the catalyst for depolymerizing a polycarbonate-based resin; adding water and an organic solvent to the depolymerization reaction product; separating a water layer containing the catalyst for depolymerizing a polycarbonate resin; and removing water contained in the water layer.

Below, a catalyst for depolymerizing a polycarbonate-based resin, a preparation method thereof, and a decomposition method of a polycarbonate-based resin, and a recovery method of a catalyst for depolymerizing a polycarbonate-based resin according to specific embodiments of the present disclosure will be described in more detail.

Unless explicitly stated herein, the technical terms used herein are for the purpose of describing specific embodiments only and is not intended to limit the scope of the invention.

The singular forms "a," "an" and "the" used herein are intended to include plural forms, unless the context clearly indicates otherwise.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

As used herein, a derivative compound means a compound in which, when an organic compound is included as a matrix, it is changed within the limit which does not significantly change the structure and properties of the matrix, such as introduction of a functional group, oxidation, reduction, or substitution of atoms.

In the present disclosure, examples of substituents are described below, but are not limited thereto.

As used herein, the term "substituted" means that other functional groups instead of a hydrogen atom in the compound are bonded, and a position to be substituted is not limited as long as the position is a position at which the hydrogen atom is substituted, that is, a position at which the substituent can be substituted, and when two or more are substituted, the two or more substituents may be the same as or different from each other.

As used herein, the term "substituted or unsubstituted" means being unsubstituted or substituted with one or more substituents selected from the group consisting of deuterium; a halogen group; a cyano group; a nitro group; a hydroxyl group; a carbonyl group; an ester group; an imide group; an amide group; a primary amino group; a carboxy group; a sulfonic acid group; a sulfonamide group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkoxysilylalkyl group; an arylphosphine group; or a heterocyclic group containing at least one of N, O, and S atoms, or being unsubstituted or substituted with a substituent to which two or more substituents are linked among the substituents exemplified above. For example, "the substituent to which two or more substituents are linked" can be a biphenyl group. That is, the biphenyl group can also be an aryl group, and can be interpreted as a substituent to which two phenyl groups are linked.

As used herein, the alkyl group is a monovalent functional group derived from an alkane, and may be a straight-chain or a branched-chain. The number of carbon atoms of the straight chain alkyl group is not particularly limited, but is

preferably 1 to 20. Also, the number of carbon atoms of the branched chain alkyl group is 3 to 20. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, 2,6-dimethylheptane-4-yl and the like, but are not limited thereto. The alkyl group may be substituted or unsubstituted, and when substituted, examples of the substituent are the same as described above.

As used herein, examples of the halogen include fluorine, chlorine, bromine, or iodine.

## 1. **Catalyst for depolymerizing a polycarbonate-based resin**

According to one embodiment of the disclosure, there can be provided a catalyst for depolymerizing a polycarbonate-based resin, comprising a compound represented by the following Chemical Formula 1:

[Chemical Formula 1]

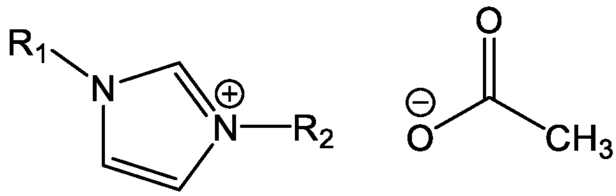

wherein in Chemical Formula 1, $R_1$ and $R_2$ are a straight chain alkyl having 1 to 3 carbon atoms.

The present inventors confirmed through experiments that the catalyst for depolymerizing a polycarbonate-based resin according to one embodiment can secure an aromatic diol compound in a high yield by recycling a polycarbonate-based resin through chemical decomposition, and can improve chemical decomposition reactivity and reaction efficiency, and the completed the present disclosure.

Specifically, the catalyst for depolymerizing a polycarbonate-based resin according to one embodiment may include the compound represented by Chemical Formula 1. The compound represented by Chemical Formula 1 is a salt composed of an imidazolium cation and an acetate anion, and can simultaneously function as both an acid catalyst and a base catalyst.

That is, the compound represented by Chemical Formula 1, which is the catalyst for depolymerizing a polycarbonate-based resin according to one embodiment, corresponds to an ionic liquid, which is a salt that maintains a liquid phase over a wide temperature range due to the structural asymmetry between ions consisting of a cation and an anion.

The acid catalyst or base catalyst conventionally used as a catalyst for depolymerizing a polycarbonate-based resin has problems in that it corrodes the equipment, and requires wastewater treatment and neutralization processes within the process, and has disadvantages in that the reaction is terminated by a neutralization reaction, and the catalyst cannot be recovered, thus making it uneconomical.

On the other hand, the compound represented by Chemical Formula 1 is an ionic liquid, in which reactor management and replacement due to reactor corrosion are not required, a separate neutralization process is not required, and the catalyst can be recycled after recovery, whereby the compound can act as a catalyst that is economical and environmentally superior to existing acid catalysts or base catalysts.

Further, the catalyst for depolymerizing a polycarbonate-based resin according to one embodiment is an ionic liquid which is a salt consisting of a cation and an anion, which simultaneously performs the roles of an acid catalyst and a base catalyst, whereby when the step of subjecting the polycarbonate-based resin to depolymerization reaction in the presence of the polycarbonate-based resin depolymerization catalyst according to one embodiment is proceeded, it is possible to improve the depolymerization reactivity.

Further, the compound represented by Chemical Formula 1 has high selective solubility and is thermally and chemically stable, enabling the depolymerization of polycarbonate-based resins in high yield and high efficiency. Specifically, the catalyst for depolymerizing a polycarbonate-based resin according to one embodiment has high solubility in polycarbonate-based resins, so that the depolymerization reaction can be proceeded sufficiently without adding any organic solvent other than alcohol solvent. Thereby, the use of organic solvents requiring a separate wastewater treatment process can be reduced, thereby increasing process efficiency.

In Chemical Formula 1, $R_1$ and $R_2$ may be a straight chain alkyl having 1 to 3 carbon atoms. That is, in Chemical Formula 1, both $R_1$ and $R_2$ are a straight chain alkyl having 1 to 3 carbon atoms. Examples of the straight chain alkyl having

1 to 3 carbon atoms include methyl, ethyl, and propyl.

In Chemical Formula 1, since $R_1$ and $R_2$ satisfy a straight chain alkyl having 1 to 3 carbon atoms and thereby have a structurally small steric effect and chemically large basicity, the depolymerization reactivity of the polycarbonate resin can be improved, the decomposition efficiency of the polycarbonate-based resin can be increased, and the yield of bisphenol A obtained by the decomposition of a polycarbonate-based resin can also be improved. In addition, when the polycarbonate-based resin is recovered and reused after the depolymerization reaction, it is recovered in high purity and recovery rate, and therefore, even if it is repeatedly reused several times, the depolymerization reactivity of the polycarbonate-based resin and the yield of bisphenol A can be maintained at a high level.

On the other hand, if at least one of $R_1$ and $R_2$ in Chemical Formula 1 is a straight chain alkyl having 4 or more carbon atoms, there may be a problem that the depolymerization reactivity of the polycarbonate-based resin and the yield of bisphenol A are reduced due to the structurally large steric effect and chemically low basicity. In addition, when the polycarbonate-based resin is recovered and reused after the depolymerization reaction, there is a limitation in that due to the decrease in purity and recovery rate, it is difficult to maintain the depolymerization reactivity of a polycarbonate-based resin and the yield of bisphenol A at a sufficiently high level when it is repeatedly reused.

Specifically, in Chemical Formula 1, $R_1$ and $R_2$ may be a straight chain alkyl having 1 to 2 carbon atoms. That is, in Chemical Formula 1, both $R_1$ and $R_2$ are a straight chain alkyl having 1 to 2 carbon atoms. Examples of the straight chain alkyl having 1 to 2 carbon atoms include methyl and ethyl.

Further, in Chemical Formula 1, $R_1$ and $R_2$ may be different from each other. One of the $R_1$ and $R_2$ may be ethyl, and the other may be methyl. That is, if the $R_1$ is ethyl, $R_2$ may be methyl. Alternatively, if the $R_1$ is ethyl, $R_2$ may be methyl.

More specifically, the compound represented by Chemical Formula 1 may be a compound represented by the following Chemical Formula 1-1.

[Chemical Formula 1-1]

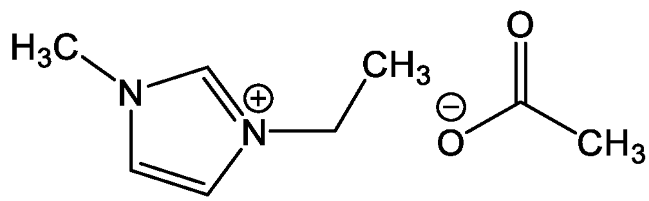

On the other hand, the compound represented by Chemical Formula 1 may be a compound represented by the following Chemical Formula 2. Alternatively, the catalyst for depolymerizing a polycarbonate-based resin may include a compound represented by the following Chemical Formula 2.

[Chemical Formula 2]

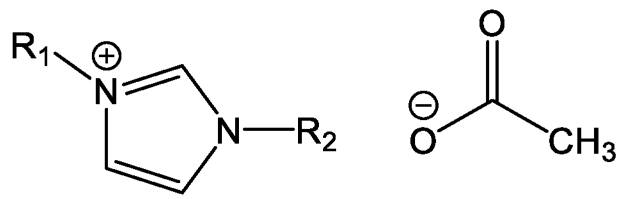

wherein in Chemical Formula 2, $R_1$ and $R_2$ include the contents set forth above in Chemical Formula 1 as they are. More specifically, the compound represented by Chemical Formula 2 may be a compound represented by the following Chemical Formula 2-1.

[Chemical Formula 2-1]

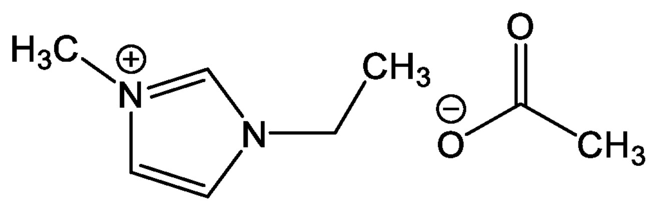

That is, the compound represented by Chemical Formula 1 and the compound represented by Chemical Formula 2 correspond to resonance structures of each other.

That is, the compound represented by Chemical Formula 1 may be a compound represented by the following Chemical Formula 3. Alternatively, the catalyst for depolymerizing a polycarbonate-based resin can include a compound represented by the following Chemical Formula 3.

[Chemical Formula 3]

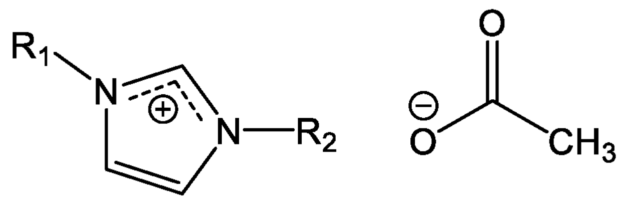

wherein in Chemical Formula 3, $R_1$ and $R_2$ include the contents set forth above in Chemical Formula 1 as they are. More specifically, the compound represented by Chemical Formula 3 may be a compound represented by the following Chemical Formula 3-1.

[Chemical Formula 3-1]

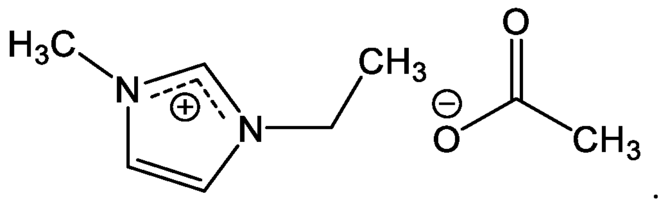

### 2. Preparation method of a catalyst for depolymerizing a polycarbonate-based resin

According to other embodiments of the disclosure, there can be provided a preparation method of a catalyst for depolymerizing a polycarbonate-based resin, the method comprising the steps of: reacting 1-alkyl-imidazole and alkyl halide to prepare a compound represented by the following Chemical Formula 4; and reacting the compound represented by Chemical Formula 4 with an alkali metal acetate:

[Chemical Formula 4]

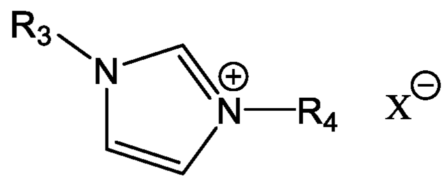

wherein in Chemical Formula 4, $R_3$ and $R_4$ are a straight chain alkyl having 1 to 3 carbon atoms, and X is halogen.

Specifically, the preparation method of a catalyst for depolymerizing a polycarbonate-based resin of the other embodiments may comprise a step of reacting 1-alkyl-imidazole and alkyl halide to prepare the compound represented by Chemical Formula 4.

The 1-alkyl-imidazole is a compound in which an alkyl is bonded to the 1st nitrogen of an imidazole compound, and can be represented by the following Chemical Formula 7.

[Chemical Formula 7]

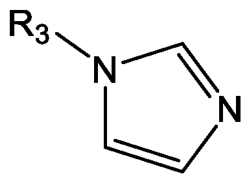

wherein in Chemical Formula 7, $R_3$ is the same as $R_3$ of Chemical Formula 4. That is, in Chemical Formula 7, the definitions of $R_3$ include all the definitions of $R_3$ of Chemical Formula 4.

Specifically, $R_3$ is a straight chain alkyl having 1 to 3 carbon atoms. Examples of the straight chain alkyl having 1 to 3 carbon atoms include methyl, ethyl, and propyl. Specifically, in Chemical Formula 7, $R_3$ is a straight chain alkyl having 1 to 2 carbon atoms. Examples of the straight chain alkyl having 1 to 2 carbon atoms include methyl and ethyl. As a more specific example, in Chemical Formula 7, $R_3$ may be methyl. That is, an example of the compound represented by Chemical Formula 7 may include 1-methyl-imidazole.

On the other hand, the alkyl halide may be represented by the following Chemical Formula 8.

[Chemical Formula 8] $R_4$-X

wherein in Chemical Formula 8, $R_4$ is the same as $R_3$ of Chemical Formula 4. That is, in Chemical Formula 8, the definitions of $R_4$ includes all the definitions of $R_4$ of Chemical Formula 4.

Specifically, $R_4$ is a straight chain alkyl having 1 to 3 carbon atoms. Examples of the straight chain alkyl having 1 to 3 carbon atoms include methyl, ethyl, and propyl. Specifically, in Chemical Formula 8, $R_4$ is a straight chain alkyl having 1 to 2 carbon atoms. Examples of the straight chain alkyl having 1 to 2 carbon atoms include methyl and ethyl. As a more specific example, in Chemical Formula 8, $R_4$ may be ethyl. In addition, in Chemical Formula 8, X is a halogen, and examples of the halogen include fluorine, chlorine, bromine, or iodine.

That is, examples of the compound represented by Chemical Formula 8 may include ethyl bromide or ethyl chloride.

A compound obtained by reacting 1-alkyl-imidazole and an alkyl halide may be represented by the following Chemical Formula 4.

[Chemical Formula 4]

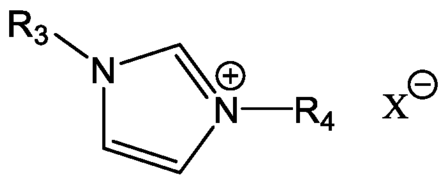

wherein in Chemical Formula 4, $R_3$ and $R_4$ are a straight chain alkyl having 1 to 3 carbon atoms, and X is halogen.

In Chemical Formula 4, $R_3$ and $R_4$ may be a straight chain alkyl having 1 to 3 carbon atoms. That is, in Chemical Formula 1, both $R_1$ and $R_2$ are a straight chain alkyl having 1 to 3 carbon atoms. Examples of the straight chain alkyl having 1 to 3 carbon atoms include methyl, ethyl, and propyl.

Specifically, in Chemical Formula 4, $R_3$ and $R_4$ may be a straight chain alkyl having 1 to 2 carbon atoms. That is, in Chemical Formula 4, both $R_3$ and $R_4$ are a straight chain alkyl having 1 to 2 carbon atoms. Examples of the straight chain alkyl having 1 to 2 carbon atoms include methyl, or ethyl.

In addition, in Chemical Formula 4, $R_3$ and $R_4$ may be different from each other. One of the $R_3$ and $R_4$ may be ethyl, and the other may be methyl. That is, if the $R_3$ is ethyl, $R_4$ may be methyl. Alternatively, if the $R_4$ is ethyl, $R_3$ may be methyl.

X is halogen, and examples of the halogen include fluorine, chlorine, bromine, or iodine.

More specifically, the compound represented by Chemical Formula 4 may be a compound represented by the following Chemical Formula 4-1, or a compound represented by the following Chemical Formula 4-2.

[Chemical Formula 4-1]

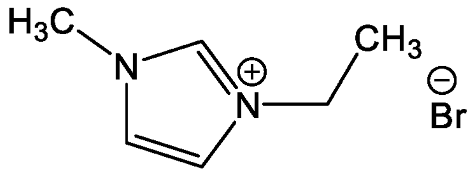

[Chemical Formula 4-2]

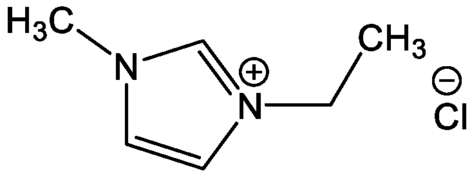

On the other hand, the compound represented by Chemical Formula 4 may be a compound represented by the following Chemical Formula 5.

[Chemical Formula 5]

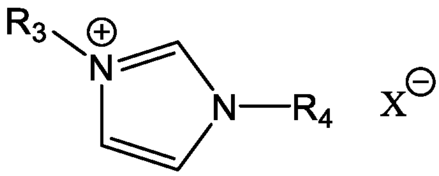

wherein in Chemical Formula 5, $R_3$, $R_4$ and X include the contents set forth above in Chemical Formula 4 as they are. More specifically, the compound represented by Chemical Formula 5 may be a compound represented by the following Chemical Formula 5-1, or a compound represented by the following Chemical Formula 5-2.

[Chemical Formula 5-1]

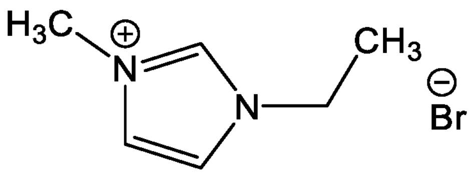

[Chemical Formula 5-2]

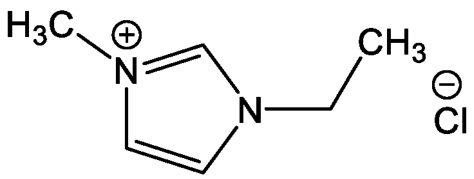

That is, the compound represented by Chemical Formula 4 and the compound represented by Chemical Formula 5 correspond to resonance structures of each other. That is, the compound represented by Chemical Formula 4 may be a compound represented by the following Chemical Formula 6.

[Chemical Formula 6]

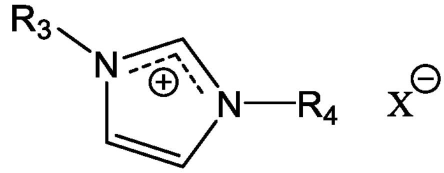

In Chemical Formula 6, $R_3$, $R_4$, and X include the contents set forth above in Chemical Formula 4. More specifically, the compound represented by Chemical Formula 6 may be a compound represented by the following Chemical Formula 6-1, or a compound represented by the following Chemical Formula 6-2.

[Chemical Formula 6-1]

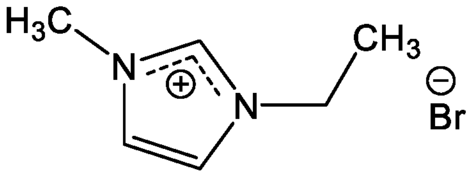

[Chemical Formula 6-2]

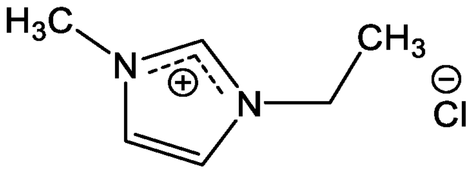

The reaction conditions under which the reaction of the 1-alkyl-imidazole with the alkyl halide is proceeded are not particularly limited, but for example, the reaction can be carried out at a temperature of 50°C to 100°C for 1 hour to 100 hours.

On the other hand, the preparation method of a catalyst for depolymerizing a polycarbonate-based resin of the other embodiments may include a step of reacting the compound represented by Chemical Formula 4 with an alkali metal acetate.

The alkali metal acetate may include potassium acetate.

The description of the compound represented by Chemical Formula 4 may include all the contents set forth above.

Examples of the reaction conditions for the step of reacting the compound represented by Chemical Formula 4 with an alkali metal acetate are not particularly limited, but may be carried out, for example, at room temperature (20~30°C) in the presence of an alcohol solvent.

On the other hand, the preparation method of the catalyst for depolymerizing a polycarbonate-based resin according to the other embodiments may further comprise a step of removing an alkali metal halide after the step of reacting the compound represented by Chemical Formula 4 with an alkali metal acetate.

When reacting the compound represented by Chemical Formula 4 with an alkali metal acetate, an alkali metal halide may be produced as a reaction byproduct. Examples of the methods for removing the alkali metal halide are not particularly limited, but include, for example, a filter removal method. Examples of the alkali metal halide are not particularly limited, but include, for example, potassium chloride(KCl) or potassium bromide(KBr).

**3. Decomposition method of a polycarbonate-based resin**

According to yet another embodiment of the present disclosure, there can be provided a decomposition method of a polycarbonate-based resin, the method comprising subjecting a polycarbonate-based resin to depolymerization reaction in the presence of the catalyst for depolymerizing a polycarbonate-based resin according to one embodiment.

Specifically, the decomposition method of a polycarbonate-based resin may comprise a step of subjecting a

polycarbonate-based resin to depolymerization reaction in the presence of a catalyst for depolymerizing a polycarbonate-based resin.

The depolymerization of the polycarbonate-based resin proceeds through an esterification reaction, wherein the esterification reaction include a method of using an acid catalyst or a method of using a base catalyst. The acid catalyst reacts so that a hydrogen cation reacts with the oxygen of the carbonyl group of a polycarbonate, which facilitates the nucleophilic attack of the alcohol on the carbonyl group. **In** addition, the base catalyst reacts so that it interacts with the hydrogen of the hydroxyl group of an alcohol to form an alkoxy group, which facilitates the nucleophilic attack on the carbonyl group of the polycarbonate.

The catalyst for depolymerizing a polycarbonate-based resin according to one embodiment is an ionic liquid that is a salt composed of a cation and an anion, which simultaneously performs the roles of an acid catalyst and a base catalyst, whereby when the step of subjecting a polycarbonate-based resin to depolymerization reaction in the presence of the catalyst for depolymerizing a polycarbonate-based resin according to one embodiment is proceeded, it is possible to improve the depolymerization reactivity.

The content of the catalyst for depolymerizing a polycarbonate-based resin includes all the contents set forth above in the one embodiment. However, the content of the catalyst may be 5 parts by weight to 90 parts by weight, or 10 parts by weight to 88 parts by weight, based on 100 parts by weight of the polycarbonate-based resin. By including the catalyst within the above content range, there is an advantage in that the catalytic reaction can proceed economically.

The polycarbonate-based resin includes one or both of homopolymers and copolymers containing polycarbonate repeating units, and refers collectively to a reaction product obtained through a polymerization reaction or copolymerization reaction of a monomer including an aromatic diol compound and a carbonate precursor. When containing one type of carbonate repeating unit obtained by using only one aromatic diol compound and one carbonate precursor, a homopolymer can be synthesized.

Further, when two or more carbonates are contained by using one aromatic diol compound and two or more carbonate precursors as the monomers, or using two or more aromatic diol compounds and one carbonate precursor, or using one or more other diols in addition to one aromatic diol compound and one carbonate precursor, a copolymer can be synthesized. The homopolymer or copolymer can include all of low-molecular-weight compounds, oligomers, and polymers according to the molecular weight range.

The polycarbonate-based resin can be applied regardless of various forms and types, such as a new polycarbonate-based resin produced through synthesis, a regenerated polycarbonate-based resin produced through a regeneration process, or a polycarbonate-based resin waste.

However, if necessary, before performing the depolymerization reaction of a polycarbonate-based resin, a pretreatment process of the polycarbonate-based resin may be performed to thereby increase the efficiency of the process of recovering the aromatic diol compound and carbonate precursor from the polycarbonate-based resin. Examples of the pretreatment process include washing, drying, pulverization, and glycol decomposition, and specific methods of each pretreatment process are not limited, and various methods that are widely used in the process of recovering the aromatic diol compound and carbonate precursor by depolymerization of the polycarbonate-based resin may be applied without limitation.

Further, the depolymerization reaction of the polycarbonate-based resin may be carried out in the presence of an alcohol solvent. More specifically, the alcohol may be ethanol. The present disclosure has advantages in that a polycarbonate-based resin can be decomposed with a solvent including ethanol to stably obtain a high-purity monomer bisphenol A, and further obtain diethyl carbonate having high added value as a reaction byproduct.

The content of the alcohol may be 50 parts by weight to 250 parts by weight based on 100 parts by weight of the polycarbonate-based resin. Since the alcohol has good solubility in bisphenol A, alcohol within the above range should essentially be included. If the content of the alcohol is excessively reduced relative to the polycarbonate-based resin, alcohol decomposition of polycarbonate-based resins is unlikely to proceed sufficiently. On the other hand, if the content of the alcohol is excessively increased relative to the polycarbonate-based resin, the economic feasibility of the process may decrease due to excessive use of alcohol.

Conventionally, the solvent in which the depolymerization reaction of the polycarbonate-based resin is proceeded has further used organic solvents such as tetrahydrofuran, toluene, methylene chloride, chloroform, dimethyl carbonate, ethyl methyl carbonate, diethyl carbonate, or dipropyl carbonate, in addition to alcohol such as ethanol, to increase the solubility of the polycarbonate-based resin.

However, in the decomposition method of a polycarbonate-based resin according to the other embodiments, since the catalyst for depolymerizing a polycarbonate-based resin according to one embodiment is used, the catalyst has high solubility in the polycarbonate-based resin, so that the depolymerization reaction can proceed sufficiently without adding any organic solvent other than an alcohol solvent. Thereby, the use of an organic solvent requiring a separate wastewater treatment process can be reduced, thereby increasing the efficiency of the process.

Meanwhile, the temperature at which the depolymerization reaction of the polycarbonate-based resin is proceeded is not particularly limited, but the reaction may be carried out, for example, at 50°C to 95°C, or 60°C to

95°C, or 85°C to 95°C. Further, the depolymerization reaction of the polycarbonate-based resin may be carried out for 1 hour to 100 hours, or 1 hour to 30 hours, or 2 hours to 24 hours.

Specifically, the above conditions are mild process conditions relative to the existing pressurized/high temperature process, and by performing stirring under the above conditions, the process can be carried out in a mild process relative to the pressurized/high temperature process. In particular, when stirring is performed at 50°C to 70°C for 4 hours to 6 hours, there is an advantage in that the most efficient results can be obtained in terms of reproducibility and recognizability.

That is, the present disclosure is advantageous in that even without using an organic catalyst, the type and amount of the mixed solvent, and the type and content of the base catalyst can be adjusted to thereby obtain a high-purity aromatic diol compound (e.g., bisphenol A) under mild conditions without using a pressurized/high-temperature process, and diethyl carbonate can be obtained as a byproduct by using an ethanol solvent.

Meanwhile, the decomposition method of a polycarbonate-based resin according to the other embodiments may further comprise a step of obtaining an aromatic diol compound, after the step of subjecting the polycarbonate-based resin to depolymerization reaction in the presence of the catalyst for depolymerizing a polycarbonate-based resin.

Specific examples of the aromatic diol compound include bis(4-hydroxyphenyl)methane, bis(4-hydroxyphenyl) ether, bis(4-hydroxyphenyl)sulfone, bis(4-hydroxyphenyl)sulfoxide, bis(4-hydroxyphenyl)sulfide, bis(4-hydroxyphenyl) ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or mixtures of two or more thereof. Preferably, the aromatic diol compound may be 2,2-bis(4-hydroxyphenyl)propane (bisphenol A).

The aromatic diol compound is characterized in that it is recovered from the polycarbonate-based resin. That is, apart from recovery from the polycarbonate-based resin, when a new aromatic diol compound is added from outside, it is not included in the category of the aromatic diol compound of the present disclosure. "Recovered from the polycarbonate-based resin" means that it is obtained through a depolymerization reaction of the polycarbonate-based resin.

The step of obtaining an aromatic diol compound may comprises a step of adding water and an organic solvent to a depolymerization reaction product; a step of separating an organic solvent layer containing an aromatic diol compound; and a step of removing an organic solvent contained in the organic solvent layer.

In the step of adding water and an organic solvent to a depolymerization reaction product, as the organic solvent, an acetate-based solvent or an ether-based solvent can be used, and more specifically, diethyl ether can be used. As water and an organic solvent are added to the depolymerization reaction product, the depolymerization reaction product can form a layer divided into a water layer containing a catalyst and an organic solvent layer containing an aromatic diol compound.

In the step of separating an organic solvent layer containing an aromatic diol compound, the organic solvent layer can be separated from the water layer in a layer divided into a water layer containing a catalyst and an organic solvent layer containing an aromatic diol compound. Specific separation conditions for separating the organic solvent layer from the water layer are not particularly limited, and as for the specific separation devices and methods, various purification techniques previously known in the art can be applied without limitations. However, as one example, a drain device can be used.

In the step of removing an organic solvent contained in the organic solvent layer, specific organic solvent removal conditions are not particularly limited, but as an example, an evaporator can be used. As for specific evaporators and methods, various evaporators known in the art can be applied without limitation. However, as an example, a rotary evaporator can be used.

The aromatic diol compound obtained above can be recycled without a separate separation and purification process, or can be recycled after undergoing separation and purification such as conventional extraction, adsorption, and drying, if necessary. Specific purification conditions are not particularly limited, and as for specific purification equipment and methods, various purification techniques previously known in the art can be applied without limitation.

In addition, if necessary, the method may further comprise a step of drying the obtained aromatic diol compound.

The remaining solvent can be removed through the drying, and specific drying conditions are not particularly limited, but for example, drying can be carried out at a temperature of 10°C to 100°C, or 10°C to 50°C. As for the specific drying equipment and method used during the drying, various known drying techniques can be applied without limitation.

### 4. Recovery method of a catalyst for depolymerizing a polycarbonate-based resin

According to yet another embodiment of the present disclosure, there can be provided a recovery method of a catalyst for depolymerizing a polycarbonate-based resin, the method comprising the steps of: subjecting a polycarbonate-based resin to depolymerization reaction in the presence of the catalyst for depolymerizing a polycarbonate-based resin

according to one embodiment; adding water and an organic solvent to the depolymerization reaction product; separating a water layer containing the catalyst for depolymerizing a polycarbonate resin; and removing water contained in the water layer.

Specifically, the recovery method of a catalyst for depolymerizing a polycarbonate-based resin according to the other embodiments may comprise a step of subjecting a polycarbonate-based resin to depolymerization reaction in the presence of the catalyst for depolymerizing a polycarbonate-based resin according to one embodiment.

The contents regarding the catalyst for depolymerizing a polycarbonate-based resin include all the contents set forth above in the one embodiment. In addition, the contents regarding the step of subjecting a polycarbonate-based resin to depolymerization reaction in the presence of the catalyst for depolymerizing a polycarbonate-based resin according to one embodiment include all the contents set forth above in the decomposition method of a polycarbonate-based resin.

The recovery method of a catalyst for depolymerizing a polycarbonate-based resin according to the other embodiments may comprise a step of adding water and an organic solvent to the depolymerization reaction product. In the step of adding water and an organic solvent to the depolymerization reaction product, as the organic solvent, an acetate-based solvent or an ether-based solvent can be used, and more specifically, diethyl ether can be used. As water and an organic solvent are added to the depolymerization reaction product, the depolymerization reaction product can form a layer divided into a water layer containing a catalyst and an organic solvent layer containing an aromatic diol compound.

The recovery method of a catalyst for depolymerizing a polycarbonate-based resin according to the other embodiments may comprise a step of separating a water layer containing the catalyst for depolymerizing a polycarbonate resin. In the step of separating a water layer containing the catalyst for depolymerizing a polycarbonate resin, the water layer can be separated from the organic solvent layer in a layer divided into a water layer containing a catalyst and an organic solvent layer containing an aromatic diol compound. Specific separation conditions for separating the water layer from the organic solvent layer are not particularly limited, and as for specific separation devices and methods, various purification techniques previously known in the art can be applied without limitation. However, as an example, a drain device can be used.

On the other hand, the recovery method of a catalyst for depolymerizing a polycarbonate-based resin according to the other embodiments may comprise a step of removing water contained in the water layer. Thereby, the catalyst for depolymerizing a polycarbonate-based resin contained in the water layer can be recovered.

Specifically, the step of removing water contained in the water layer may comprise a step of freeze-drying water. The step of freeze-drying water may be carried out at a temperature of -90°C to -70°C for 1 hour to 100 hours. The catalyst recovered by such freeze-drying can have high purity even when reused several times in the decomposition reaction of a polycarbonate, and thus the decomposition rate of a polycarbonate can be maintained at a high level.

**[Advantageous Effects]**

According to the present disclosure, a catalyst for depolymerizing a polycarbonate-based resin and a preparation method thereof, which can secure an aromatic diol compound in a high yield by recycling a polycarbonate-based resin through chemical decomposition, and can improve chemical decomposition reactivity and reaction efficiency, and a decomposition method of a polycarbonate-based resin by using the same and a recovery method of catalyst for depolymerizing a polycarbonate-based resin can be provided.

**[DETAILED** DESCRIPTION OF THE EMBODIMENTS]

Hereinafter, exemplary embodiments of the present disclosure will be described in more detail with reference to the following examples. However, the following examples are provided for illustrative purposes only, and the scope of the present disclosure is not intended to be limited thereby.

<Synthesis Example and Comparative Synthesis Example: Preparation of Catalyst>

<u>Synthesis Example 1</u>

1-Methyl imidazole and ethyl bromide were added to a 20 ml vial, and stirred at 70°C for 24 hours. When the reaction was completed according to the following Reaction Scheme 1, Compound 1 as a pale yellow liquid was obtained.

[Reaction Scheme 1]

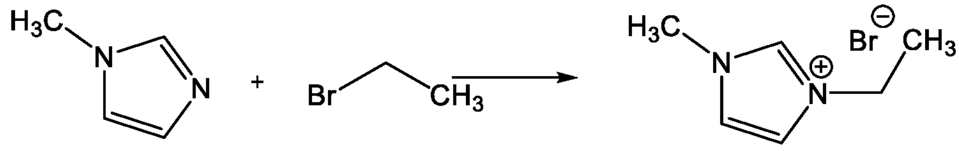

1

The Compound 1 and potassium acetate were added to methanol by the same number of moles and stirred. The KBr salt produced according to the following Reaction Scheme 2 was removed by a filter to obtain Compound 2 (1-ethyl-3-methylimidazolium acetate ([EMIM][Ac])) as a pale yellow liquid.

[Reaction scheme 2]

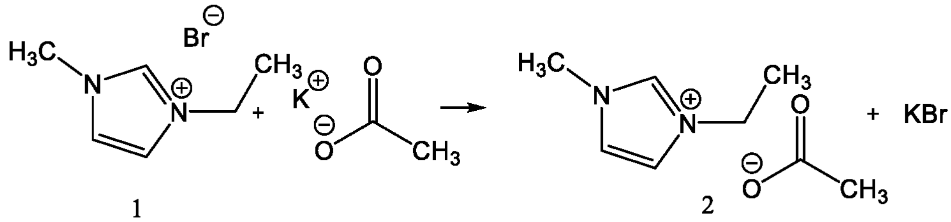

1 2

Synthesis Example 2

Compound 3 was obtained according to the following Reaction Scheme 3 in the same manner as in Synthesis Example 1, except that ethyl chloride was used instead of ethyl bromide in Synthesis Example 1.

[Reaction scheme 3]

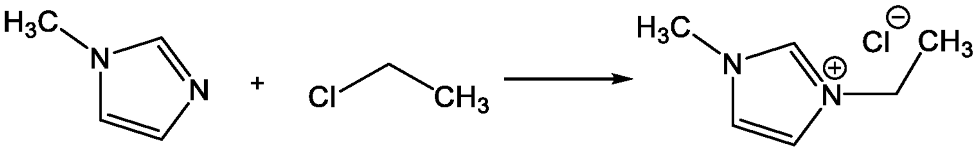

3

The Compound 3 and potassium acetate were added to methanol by the same number of moles and stirred. The KCl salt produced according to the following Reaction Scheme 4 was removed by a filter to obtain Compound 4 (1-ethyl-3-methylimidazolium acetate, [EMIM][Ac])) as a pale yellow liquid.

[Reaction Scheme 4]

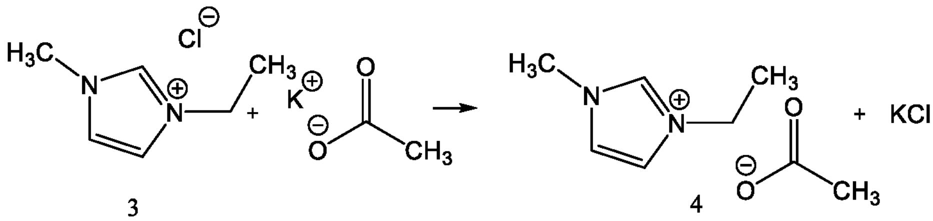

3 4

Comparative Synthesis Example 1

Compound 5 was obtained according to the following Reaction Scheme 5 in the same manner as in Synthesis Example 1, except that 1-n-butyl bromide was used instead of ethyl bromide in Synthesis Example 1.

[Reaction Scheme 5]

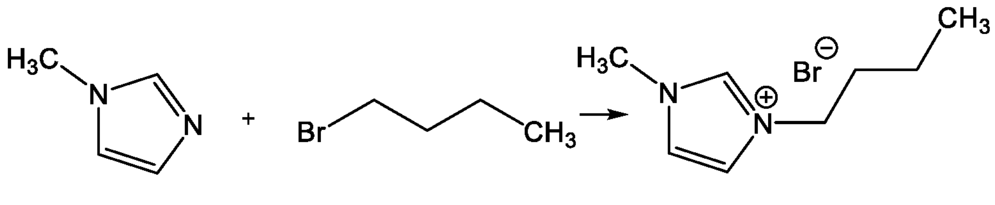

5

The Compound 5 and potassium acetate were added to methanol by the same number of moles and stirred. The KBr salt produced according to the following Reaction Scheme 6 was removed by a filter to obtain Compound 6 (1-n-butyl-3-methylimidazolium acetate, [BMIM][Ac]) as a pale yellow liquid.

[Reaction Scheme 6]

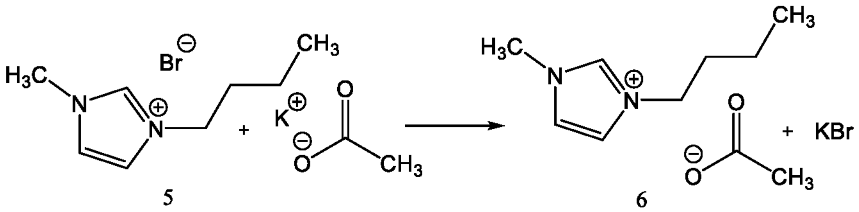

5 6

**<Example and Comparative Example>**

Example 1

(1) Decomposition of polycarbonate

In a 20 ml vial, polycarbonate(PC) and the catalyst[EMIM][Ac] obtained in Synthesis Example 1 or Synthesis Example 2 were added in a ratio of Polycarbonate:[EMIM][Ac] = 2:1(weight ratio), and EtOH was added in an amount of 50 wt.% relative to PC, and the mixture was stirred at a reaction temperature of 90°C for 10 hours to proceed a depolymerization reaction

(2) Obtaining bisphenol A

Water (1~ 5 equivalents) and diethyl ether were added to the solution where the depolymerization reaction was completed. At this time, the catalyst [EMIM][Ac] was separated into a water layer, and the depolymerization reaction product, i.e., bisphenol A (BPA), was separated into a diethyl ether layer, so that the water layer and the diethyl ether layer were separated. Bisphenol A contained in the separated diethyl ether layer was obtained using a rotary evaporator.

(3) Recovery of catalyst

The water layer separated in step (2) was freeze-dried at a temperature of -80°C for 72 hours to remove water, and the [EMIM][Ac] catalyst was recovered.

Examples 2 to 15, Comparative Examples 1 to 3

(1) Decomposition of polycarbonate

The depolymerization reaction was carried out in the same manner as in (1) of Example 1, except that the depolymerization reaction conditions were changed as described in Table 1 below.

(2) Obtaining bisphenol A

Bisphenol A was obtained in the same manner as in (2) of Example 1.

(3) Recovery of catalyst

The catalyst was recovered in the same manner as in (3) of Example 1.

**<Experimental Example 1>**

**1. Decomposition of PC**

The degree of decomposition of polycarbonate was confirmed by sampling a part of the decomposed reactants and measuring it by $^1$H NMR. Specifically, after the start of the reaction, the amount of unreacted material that could be produced during the reaction process at regular time intervals (about 2 hours) for a total of 24 hours was measured by $^1$H NMR.

**2. Yield of bisphenol A (BPA)**

The weight of BPA produced when the polycarbonate used in the reaction was 100% decomposed was measured, and the weight of the resulting BPA was measured to calculate the yield of BPA according to the following Equation 1.

[Equation 1]

$$\text{Yield (\%)} = (W_1/W_0) \text{ X } 100 \text{ (\%)}$$

wherein in Equation 1, $W_0$ is the mass of BPA obtained at 100% decomposition, and $W_1$ is the mass of BPA actually obtained. Specifically, when about 100 g of polycarbonate was decomposed, the mass of BPA theoretically obtained at 100% decomposition was 89 g. If the mass of BPA actually obtained was 80 g, the yield was (80/89) * 100 = 90%.

[Table 1]

| Measurement results of Experimental Example 1 | | | | | | | |
|---|---|---|---|---|---|---|---|
| Category | Catalyst | Solven t | PC:catalyst:solven t (weigh ratio) | Reaction temperatur e (°C) | Reactio n time (hr) | PC decompositio n | BP A yiel d (%) |
| Example 1 | [EMIM] [Ac ] | EtOH | 2:1:1 | 90 | 10 | 100 | 96.5 |
| Example 2 | [EMIM] [Ac ] | EtOH | 2:1:2 | 90 | 10 | 100 | 98.6 |
| Example 3 | [EMIM] [Ac ] | EtOH | 2:1:3 | 90 | 10 | 100 | 99.9 |
| Example 4 | [EMIM] [Ac ] | EtOH | 2:1:4 | 90 | 10 | 100 | 99.9 |
| Example 5 | [EMIM] [Ac ] | EtOH | 2:1:5 | 90 | 10 | 100 | 99.9 |
| Example 6 | [EMIM] [Ac ] | EtOH | 2:0.2:3 | 90 | 10 | 83.7 | 96.4 |
| Example 7 | [EMIM] [Ac ] | EtOH | 2:0.5:3 | 90 | 10 | 91.0 | 90.1 |
| Example 8 | [EMIM] [Ac ] | EtOH | 2:1.25:3 | 90 | 10 | 100 | 99.9 |
| Example 9 | [EMIM] [Ac ] | EtOH | 2:1.5:3 | 90 | 10 | 100 | 99.9 |
| Example 10 | [EMIM] [Ac ] | EtOH | 2:1.75:3 | 90 | 10 | 100 | 99.9 |

(continued)

| Measurement results of Experimental Example 1 | | | | | | | |
|---|---|---|---|---|---|---|---|
| Category | Catalyst | Solven t | PC:catalyst:solven t (weigh ratio) | Reaction temperatur e (°C) | Reactio n time (hr) | PC decompositio n | BP A yiel d (%) |
| Example 11 | [EMIM] [Ac ] | EtOH | 2:1:3 | 90 | 2 | 19.7 | 99.8 |
| Example 12 | [EMIM] [Ac ] | EtOH | 2:1:3 | 90 | 6 | 100 | 96.9 |
| Example 13 | [EMIM] [Ac ] | EtOH | 2:1:3 | 90 | 14 | 100 | 99.9 |
| Example 14 | [EMIM] [Ac ] | EtOH | 2:1:3 | 90 | 18 | 100 | 99.9 |
| Example 15 | [EMIM] [Ac ] | EtOH | 2:1:3 | 90 | 24 | 100 | 99.9 |
| Comparativ e Example 1 | [BMIM] [Ac ] | EtOH | 2:1:3 | 90 | 2 | 12.5 | 14.6 |
| Comparativ e Example 2 | [BMIM] [Ac ] | EtOH | 2:1:3 | 90 | 6 | 94.4 | 96.9 |
| Comparativ e Example 3 | [BMIM] [Ac ] | EtOH | 2:1:3 | 90 | 10 | 99.5 | 98.8 |

As shown in Table 1, it could be confirmed that Example 3 showed a PC decomposition of 100% and a BPA yield of 99.9%, whereas Comparative Example 3, in which only the catalyst was changed to [BMIM][Ac] under the same reaction conditions, showed a PC decomposition of 99.5% and a BPA yield of 98.8%, which were worse than those of Example 3.

Under the same principle, when comparing Example 11 with Comparative Example 1, or Example 12 with Comparative Example 2, the Comparative Example in which only the catalyst was changed to [BMIM][Ac] under the same reaction conditions showed worse results than in Examples.

**<Experimental Example 2>**

**1. Performance evaluation of the recovered catalyst**

For the [EMIM][Ac] catalyst recovered in Example 3 and the [BMIM][Ac] catalyst recovered in Comparative Example 3, the polycarbonate decomposition and catalyst recovery were repeated a total of 5 times, and the following physical properties were measured for each catalyst reuse cycle to evaluate the catalyst performance. The process of repeating the polycarbonate decomposition and catalyst recovery a total of 5 times was repeated in the same manner as in Example 3 for the catalyst recovered in Example 3, and in the same manner as in Comparative Example 3 for the catalyst recovered in Comparative Example 3.

(1) Decomposition of PC

The degree of decomposition of polycarbonate was confirmed by sampling a part of the reactants whose decomposition was completed and measuring it by $^1$H NMR. Specifically, after the start of the reaction, the amount of unreacted material that could be produced during the reaction process at regular time intervals (about 2 hours) for a total of 24 hours was measured by $^1$H NMR.

(2) Yield of Bisphenol A (BPA)

The weight of BPA produced when the polycarbonate used in the reaction was 100% decomposed was measured, and the weight of the resulting BPA was measured to calculate the yield of BPA according to the following Equation 1.

[Equation 1]

$$\text{Yield (\%)} = (W_1/W_0) \times 100(\%)$$

wherein in Equation 1, $W_0$ is the mass of BPA obtained at 100% decomposition, and $W_1$ is the mass of BPA actually obtained. Specifically, when about 100 g of polycarbonate was decomposed, the mass of BPA theoretically obtained at 100% decomposition was 89 g. If the mass of BPA actually obtained was 80 g, the yield was (80/89) * 100 = 90%.

(3) Catalyst recovery rate

The catalyst recovery rate was calculated by dividing the weight of the catalyst recovered after the reaction by the weight of the catalyst added before the reaction and multiplying the result value by 100.

(4) Catalyst purity

The recovered catalyst was diluted with DMSO-d6 to a concentration of 10 mg/ml to prepare a sample, and the NMR spectrum detected through a 1H NMR device was obtained. The area ratio of the catalyst peak among the catalyst peak and the bisphenol A (BPA) peak was measured, and used as the catalyst purity.

Specifically, the catalyst was targeted at peaks at 9.9~9.6 ppm (s, 1H) and 7.8 : 7.7 ppm (t, 1H) for [EMIM][Ac], peaks at 9.6~9.5 ppm (s, 1H), 7.8 : 7.7 (t, 1H) ppm for [BMIM][Ac], and peaks at 6.67 ppm (d, 4CH) for bisphenol A (BPA), and the catalyst purity was calculated using the following Equation 2.

Catalyst purity(%) = {(Peak area of catalyst) / 3 X 100} / (Peak area of catalyst / 3 + peak area of BPA / 4). [Equation 2]

[Table 2]

| Measurement results of Experimental Example 2 | | | | | |
|---|---|---|---|---|---|
| Category | Number of reuses | PC decomposition (%) | BPA yield (%) | Catalyst recovery rate (%) | Catalyst purity (%) |
| Example 3 | 1 | 100 | 97.6 | 100 | 99 |
| | 2 | 100 | 96.6 | 99.9 | 99 |
| | 3 | 100 | 96.2 | 99.9 | 99 |
| | 4 | 100 | 96.3 | 99.9 | 99 |
| | 5 | 100 | 95.2 | 99.9 | 99 |
| Comparative Example 3 | 1 | 99.5 | 95.08 | 99.7 | 99 |
| | 2 | 100 | 93.26 | 99.7 | 98 |
| | 3 | 100 | 88.98 | 99.3 | 98 |
| | 4 | 100 | 90.16 | 99.5 | 99 |
| | 5 | 100 | 90.14 | 98.3 | 99 |

As shown in Table 2, it could be confirmed that Example 3 had a high bisphenol A yield of 95.2% and a high catalyst recovery rate of 99.9% even when the catalyst was reused five times.

On the other hand, it could be confirmed that Comparative Example 3 had a bisphenol A yield of 90.14%, which was lower than in Example 3, and a catalyst recovery rate of 98.3%, which was lower than in Example 3, when the catalyst was reused five times.

## Claims

1. A catalyst for depolymerizing a polycarbonate-based resin, comprising a compound represented by the following Chemical Formula 1:

[Chemical Formula 1]

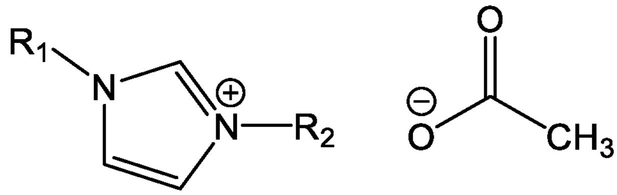

wherein in Chemical Formula 1, $R_1$ and $R_2$ are a straight chain alkyl having 1 to 3 carbon atoms.

2. The catalyst for depolymerizing a polycarbonate-based resin as set forth in claim 1, wherein: the $R_1$ and $R_2$ are a straight chain alkyl having 1 to 2 carbon atoms.

3. The catalyst for depolymerizing a polycarbonate-based resin as set forth in claim 1, wherein: the $R_1$ and $R_2$ are different from each other.

4. The catalyst for depolymerizing a polycarbonate-based resin as set forth in claim 1, wherein: one of the $R_1$ and $R_2$ is ethyl, and the other is methyl.

5. The catalyst for depolymerizing a polycarbonate-based resin as set forth in claim 1, wherein: the compound represented by Chemical Formula 1 is a compound represented by the following Chemical Formula 1-1:

[Chemical Formula 1-1]

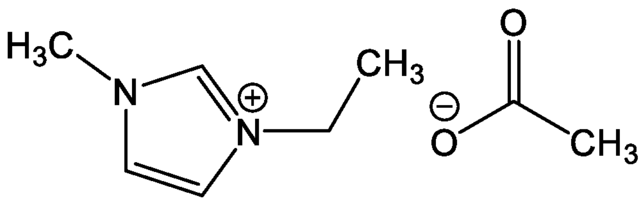

6. A preparation method of a catalyst for depolymerizing a polycarbonate-based resin, the method comprising the steps of:

reacting 1-alkyl-imidazole and alkyl halide to prepare a compound represented by the following Chemical Formula 4; and
reacting the compound represented by Chemical Formula 4 with an alkali metal acetate:

[Chemical Formula 4]

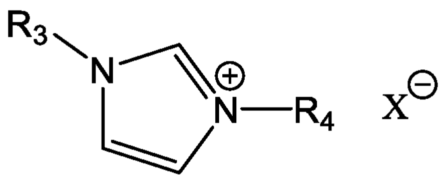

wherein in Chemical Formula 4, $R_3$ and $R_4$ are a straight chain alkyl having 1 to 3 carbon atoms, and X is halogen.

7. The preparation method of a catalyst for depolymerizing a polycarbonate-based resin as set forth in claim 6, wherein:

the 1-alkyl-imidazole is a compound represented by the following Chemical Formula 7:

[Chemical Formula 7]

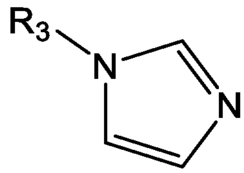

wherein in Chemical Formula 7, $R_3$ is the same as $R_3$ of Chemical Formula 4.

**8.** The preparation method of a catalyst for depolymerizing a polycarbonate-based resin as set forth in claim 6, wherein:

the alkyl halide is a compound represented by the following Chemical Formula 8:

[Chemical Formula 8] $R_4$-X

wherein in Chemical Formula 8, $R_4$ is the same as $R_3$ of Chemical Formula 4, and X is halogen.

**9.** The preparation method of a catalyst for depolymerizing a polycarbonate-based resin as set forth in claim 6, further comprising removing an alkali metal halide, after the reacting the compound represented by Chemical Formula 4 with an alkali metal acetate.

**10.** A decomposition method of a polycarbonate-based resin, the method comprising subjecting a polycarbonate-based resin to depolymerization reaction in the presence of the catalyst for depolymerizing a polycarbonate-based resin as set forth in claim 1.

**11.** The decomposition method of a polycarbonate-based resin as set forth in claim 10, wherein:
the content of the catalyst is 5 parts by weight to 90 parts by weight based on 100 parts by weight of the polycarbonate-based resin.

**12.** The decomposition method of a polycarbonate-based resin as set forth in claim 10, wherein:
the depolymerizing a polycarbonate-based resin is carried out in the presence of an alcohol solvent.

**13.** The decomposition method of a polycarbonate-based resin as set forth in claim 12, wherein:
the content of the alcohol is 50 parts by weight to 250 parts by weight based on 100 parts by weight of the polycarbonate-based resin.

**14.** The decomposition method of a polycarbonate-based resin as set forth in claim 12, wherein:
the alcohol is ethanol.

**15.** The decomposition method of a polycarbonate-based resin as set forth in claim 10, wherein:
the depolymerizing a polycarbonate-based resin is carried out at a temperature of 50°C to 95°C for 1 hour to 100 hours.

**16.** The decomposition method of a polycarbonate-based resin as set forth in claim 10,
further comprising obtaining an aromatic diol compound, after the subjecting a polycarbonate-based resin to depolymerization reaction in the presence of the catalyst for depolymerizing a polycarbonate-based resin.

**17.** The decomposition method of a polycarbonate-based resin as set forth in claim 16, wherein:

the obtaining an aromatic diol compound comprises,
adding water and an organic solvent to a depolymerization reaction product;
separating an organic solvent layer containing an aromatic diol compound; and
removing an organic solvent contained in the organic solvent layer.

**18.** A recovery method of a catalyst for depolymerizing a polycarbonate-based resin, the method comprising the steps of:

subjecting a polycarbonate-based resin to depolymerization reaction in the presence of the catalyst for depolymerizing a polycarbonate-based resin as set forth in claim 1;
adding water and an organic solvent to the depolymerization reaction product;

separating a water layer containing the catalyst for depolymerizing a polycarbonate resin; and
removing water contained in the water layer.

**19.** The recovery method of a catalyst for depolymerizing a polycarbonate-based resin as set forth in claim 18, wherein: the removing water contained in the water layer comprises freeze-drying water.

**20.** The recovery method of a catalyst for depolymerizing a polycarbonate-based resin as set forth in claim 19, wherein: the freeze-drying water is carried out at a temperature of -90°C to -70°C for 1 hour to 100 hours.

**INTERNATIONAL SEARCH REPORT**

International application No. **PCT/KR2024/009452**

**A. CLASSIFICATION OF SUBJECT MATTER**

**B01J 31/02**(2006.01)i; **B01J 31/04**(2006.01)i; **B01J 37/04**(2006.01)i; **B01J 38/48**(2006.01)i; **C08J 11/28**(2006.01)i; **C07C 37/055**(2006.01)i; **C07C 37/70**(2006.01)i; **C07C 39/16**(2006.01)i

According to International Patent Classification (IPC) or to both national classification and IPC

**B. FIELDS SEARCHED**

Minimum documentation searched (classification system followed by classification symbols)

B01J 31/02(2006.01); C07C 37/00(2006.01); C07C 37/055(2006.01); C07C 39/16(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 폴리카보네이트(polycarbonate), 해중합(depolymerization), 이미다졸리움(imidazolium), 아세테이트(acetate), 회수(recover)

**C. DOCUMENTS CONSIDERED TO BE RELEVANT**

| Category* | Citation of document, with indication, where appropriate, of the relevant passages | Relevant to claim No. |
|---|---|---|
| Y | LIU, F. et al. Methanolysis of polycarbonate catalysed by ionic liquid [Bmim][Ac]. Journal of hazardous materials. 2011, vol. 189, pp. 249-254.<br>See abstract; pages 249-251; and table 3. | 1-20 |
| Y | CN 101429100 A (QINGDAO UNIVERSITY OF SCIENCE AND TECHNOLOGY) 13 May 2009 (2009-05-13)<br>See claims 2, 3 and 7; and page 5. | 1-20 |
| Y | HUANG, W. et al. Degradation of polycarbonate to produce bisphenol A catalyzed by imidazolium-based DESs under metal-and solvent-free conditions. RSC advances. 2021, vol. 11, pp. 1595-1604.<br>See abstract; and page 1597. | 16-20 |
| Y | CN 108607604 A (QINGDAO UNIVERSITY OF SCIENCE AND TECHNOLOGY) 02 October 2018 (2018-10-02)<br>See claims 1-8. | 16-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 October 2024** | **07 October 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2024/009452**

**C. DOCUMENTS CONSIDERED TO BE RELEVANT**

| Category* | Citation of document, with indication, where appropriate, of the relevant passages | Relevant to claim No. |
|---|---|---|
| PX | SEO, S. et al. Polycarbonate ethanolysis with imidazolium-based ionic liquids: Recovery of high-purity BPA and value-added by-product. Materials today sustainability. 2024 [electronic publication: 02 January 2024], vol. 25, thesis no.: 100665, inner pp. 1-9. See abstract; and inner pages 2-7. | 1-20 |

Form PCT/ISA/210 (second sheet) (July 2022)

**INTERNATIONAL SEARCH REPORT**
**Information on patent family members**

International application No.
**PCT/KR2024/009452**

| Patent document cited in search report | Publication date (day/month/year) | Patent family member(s) | Publication date (day/month/year) |
|---|---|---|---|
| CN 101429100 A | 13 May 2009 | CN 101429100 B | 23 May 2012 |
| CN 108607604 A | 02 October 2018 | CN 108607604 B | 31 March 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020230099505